# EUROPÄISCHE PATENTANMELDUNG

(11) **EP 2 471 486 A2**
(43) Veröffentlichungstag der Anmeldung: **04.07.2012**
(21) Anmeldenummer: 11192056.7
(22) Anmeldetag: 06.12.2011
(51) Int. Cl.: A61C 13/00, A61C 9/00, A61C 7/00

(54) **Registrierung von 3D-Messdaten von Kiefermodellen in einem schaedelbasisbezogenen Koordinatensystem mit Hilfe eines computergestuetzten Registriersystems**

(30) Priorität: 06.12.2010 EP 10015321; 22.03.2011 EP 11002329
(71) Anmelder: Lampalzer, Ralf, 44789 Bochum (DE); Fuhrmann, Frank, 59519 Moehnesee (DE); Friemel, Jörg, 44892 Bochum (DE)
(72) Erfinder: Lampalzer, Ralf, 44789 Bochum (DE)
(74) Vertreter: Leonhard, Frank Reimund

(57) **Zusammenfassung**

Der Erfindung liegt die Aufgabenstellung zu Grunde, die räumliche Beziehung zwischen Schädel und den Messdaten von Kiefermodellen, wie sie mit optischen Scannern gewonnen werden, herzustellen. Dies geschieht durch Bestimmung der räumlichen Beziehung zwischen paraokklusalem Löffel bzw. Bissgabel und Kiefergelenken mittels eines computergestützten Registriersystems und Bestimmung der räumlichen Beziehung zwischen paraokklusalem Löffel bzw. Bissgabel und Kiefermodell durch Aufbringen lokalisierbarer Strukturen, deren räumliche Beziehung zum paraokklusalen Löffel bzw. zur Bissgabel bekannt ist.

## Beschreibung

Die vorliegende Erfindung betrifft allgemein das Gebiet der Kieferorthopädie und der zahnärztlichen Prothetik und zu gehörige Verfahren und Vorrichtungen.

Für die Kieferorthopädie und die zahnärztliche Prothetik werden Kiefermodelle mit optischen 3D-Scannern vermessen und ihre Form digital erfasst.

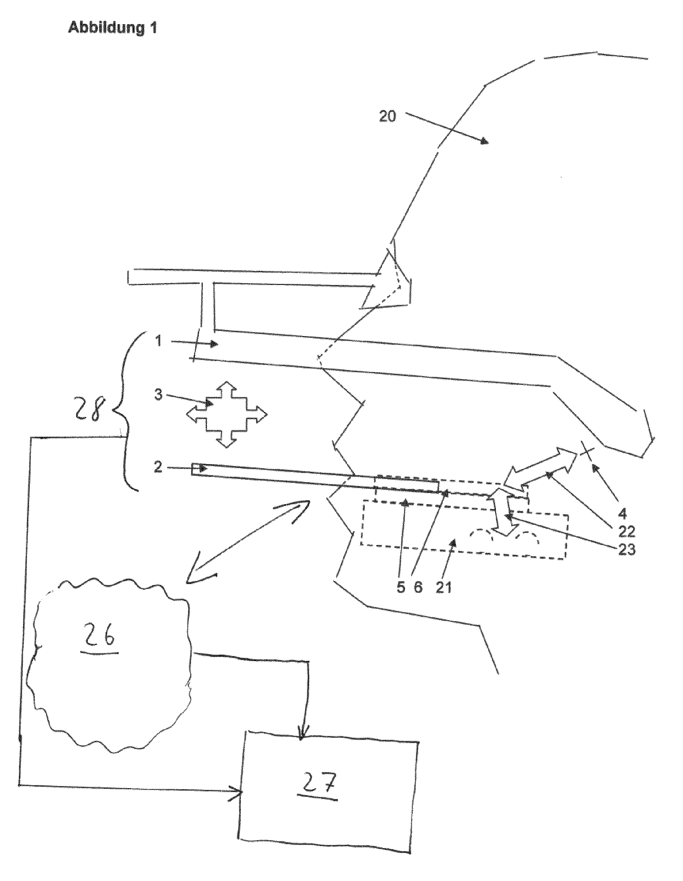

Sowohl in der Kieferorthopädie als auch in der zahnärztlichen Prothetik wird der Bezug dieser Daten zum Schädel 20 benötigt, wie dies schematisch in Figur 1 gezeigt ist. Insbesondere ist die Lage der Kiefergelenke 4 relativ zu den Daten der Kiefermodelle von Bedeutung. Das menschliche Kausystem funktioniert durch das Zusammenspiel der Oberflächenform der Zähne (Höcker, Fissuren) mit der Bewegung der Kiefergelenke. In der zahnärztlichen Prothetik kann deswegen die Oberflächenform von Zahnersatz computergestützt besser berechnet werden, wenn die Lage der Kiefergelenke bekannt ist. In der Kieferorthopädie kann eine verbesserte Diagnose ("Funktionsdiagnose") und Therapie durchgeführt werden, wenn die Lage der Kiefergelenke bekannt ist.

### Vorbemerkungen zur Begriffswahl

- Die Kiefergelenke sind von einer komplizierten Form, welche zur Beschreibung der Erfindung nicht erforderlich ist. Mit dem Begriff "Ort des Kiefergelenks" soll eine mittlere anatomische Stellung des Kiefergelenks gemeint sein, von der aus das Kiefergelenk aufgrund seiner Anatomie mindestens einen, bevorzugt mehrere Bewegungsfreiheitsgrad bzw. Bewegungsfreiheitsgrade hat.
- Mit dem Begriff "räumliche Beziehung zwischen zwei Objekten" ist folgendes gemeint. In jedem Objekt werden ein Koordinatensystem und ein Koordinatenursprung definiert. Die räumliche Beziehung zwischen den Objekten ist mathematisch die Transformation der Koordinatensysteme von einem Objekt zum anderen Objekt. Sie besteht aus Translation und Drehung, kann z.B. durch einen Translationsvektor mit drei Komponenten und drei Eulerwinkel ausgedrückt werden.

Häufig werden, sowohl in der Kieferorthopädie als auch in der zahnärztlichen Prothetik, der Zahnersatz oder kieferorthopädische Apparaturen nicht voll computergestützt hergestellt, sondern in Handarbeit oder mit manuellen Teilschritten oder manuellen Kontrollschritten.

Dazu werden die Kiefermodelle in einen Artikulator eingesetzt. Ein Artikulator ist ein Apparat, der die Kiefergelenke durch mechanische Gelenke (die zusätzlichen Spielraum für typische Kieferbewegungen haben) nachgebildet.

Damit die Kiefermodelle am richtigen Ort in den Artikulator eingesetzt werden, wird der Gesichtsbogen abgenommen. Nimmt man den Gesichtsbogen ab, so verkörpert er mechanisch die räumliche Beziehung zwischen Kiefergelenken und der Bezahnung. Mit Hilfe dieser mechanisch verkörperten räumlichen Beziehung werden die Modelle im Artikulator platziert. Diesen Vorgang nennt man "Einartikulieren der Modelle". Zur Platzierung dient in der Regel eine Portion Gips, welche zwischen die Modelle und Sockelplatten des Artikulators gegeben wird. Das System bestehend aus Artikulator und Kiefermodellen bildet dann das Kausystem mechanisch nach. Die artikulierten Modelle kann man aus dem Artikulator entnehmen, lagern und reproduzierbar wieder einsetzen.

Es besteht häufig der Bedarf, eine räumliche Beziehung zwischen Schädel und den Messdaten von Kiefermodellen, wie sie mit optischen Scannern gewonnen werden, herzustellen.

DE10 2008 060 504A1, AT507 887 B1, und EP1 105 067B1lösen diese Problematik durch Übertragung der artikulierten Modelle aus dem Artikulator in einen optischen 3D-Scanner, welcher einen Sockel im Scanner aufweist, der zu dem Sockel im Artikulator baugleich ist. Die Lage des Sockels im Scanner ist im Koordinatensystem des 3D-Scanners bekannt. Da der Sockel im Artikulator eine bekannte schädelbezogene Position hat, kann die Lage der Gelenke des Artikulators relativ zu den Messdaten der Kiefermodelle berechnet werden.

Diese Vorgehensweise ist zeitraubend und in gewisser Hinsicht problematisch.

Der Scanner muss Kiefermodelle aufnehmen können, welche auf einem Artikulatorsockel befestigt sind. Diese Objekte sind relativ groß und schwer und haben eine relativ große Variation in der Lage des Modells relativ zum Sockel.

Der notwendige Scanner benötigt (i) einen größeren Innenraum, und (ii) im Allgemeinen eine kräftigere Bewegungsmechanik für die großen und schweren einartikulierten Modelle.

In dieser Vorgehensweise, die im Folgenden erläutert wird, wird ein "computergestütztes Registriersystem" verwendet. Ein computergestütztes Registriersystem ist eine Fortentwicklung eines Gesichtsbogens.

Als Produkte sind z.B. folgende computergestützte Registriersysteme erhältlich: **Axioquick** (SAM Präzisionstechnik), **Arcus Digma** (KaVo), **Cardiax** (Girrbach), **Condylocomp** (Dentron), **Freecorder** (DDI Group). Ein Registriersystem zeigt auch Schulz, Winzen, "Basiswissen zur Datenübertragung", teamwork media, 2004, ISBN 3-932599-16-0.

Weiterhin mit Bezug zu Fig. 1 sei eine diesbezügliche Vorgehensweise unter Verwendung eines computergestützten Registriersystems 28schematisch beschrieben, wobei wir folgende Definitionen vereinbaren: der obere Teil des elektronischen Registriersystems ist über Weichgewebe oder über die Zähne des Oberkiefers ausreichend starr mit dem Schädel 20 verbunden. Er soll "schädelbezogener Komponente", die mit 1 bezeichnet ist, heißen.

Der untere Teil des computergestützten Registriersystems soll als "unterkieferbezogene Komponente" 2 bezeichnet werden. Diese ist mit dem Unterkiefer verbunden. Diese Verbindung geschieht mit Hilfe eines paraokklusal am Unterkiefer anhaftenden Löffels 5. Der paraokklusale Löffel 5 wird mit Hilfe eines Bissregistriermaterials 6, eines Composites, oder eines anderen Materials am Unterkiefer befestigt. Der paraokklusale Löffel 5 ist mit einem mechanischen Interface, d.h. einem Adapter zur unterkieferbezogenen Komponente, 8 von der unterkieferbezogenen Komponente trennbar.

Bei manchen, aber nicht bei allen elektronischen Registriersystemen (z.B. Arcus Digma) kann die unterkieferbezogene Komponente zu einem früheren oder späteren Zeitpunkt zusätzlich auch noch mit dem Oberkiefer verbunden werden. Dies geschieht mit einer Bissgabel, die hier ebenfalls mit dem Bezugszeichen 5 bezeichnet ist und mit Hilfe von Abdruckmasse oder einem anderen Material am Oberkiefer befestigt wird. Die Bissgabel muss, ebenso wie der paraokklusalen Löffel zu dem Adapter zur unterkieferbezogenen Komponente 8 passen, und wird dort im Austausch zum paraokklusalen Löffel eingesteckt.

Das elektronische Registriersystem 28, das also zumindest die Komponenten 1 und 2 umfasst, verfügt über eine Messmethode 3, ("Messmethode des elektronischen Registriersystems"), um die Lage der unterkieferbezogenen Komponente 1 relativ zur oberkieferbezogenen Komponente 2 in Raum- und Winkelfreiheitsgraden zu erfassen. Diese Messmethode ist bei den beschriebenen Produkten durch Ultraschall-Laufzeitmessung oder durch optische Messungen realisiert. Somit wird das elektronische bzw. computergestützte Registriersystem zumindest durch die Komponenten 1, 2 und 3 implementiert.

Aus der computergestützten Analyse der Bewegungen der unterkieferbezogenen Komponente 1 relativ zur schädelbezogenen Komponente 2 wird beim computergestützten Registriersystem die Lage der Kiefergelenke 4, typischerweise mit den zuvor erwähnten weiteren Freiheitsgraden der Kiefergelenke) im Koordinatensystem der schädelbezogenen Komponente 1 berechnet.

Es sind zwei Verfahren bekannt, um die räumliche Beziehung zum Oberkiefer zu ermitteln.
A. Beim Registriersystem "Freecorder" erfolgt eine Null-Datenaufzeichnung mit der Messmethode 3 in Bisssituation. Damit ist eine "Nullstellung" des Unterkiefers aufgezeichnet. Die Lage des Oberkiefers in dieser Bissposition kann aus einem Bissregistrat entnommen werden.
B. Bei solchen Registriersystemen, die, wie oben erwähnt, die Möglichkeit haben, die unterkieferbezogenen Komponente 2 auch mit dem Oberkiefer zu verbinden, erfolgt die Ermittlung der räumlichen Beziehung zum Oberkiefer, indem die unterkieferbezogene Komponente 2 mit Hilfe der erwähnten Bissgabel mit dem Oberkiefer verbunden wird und eine Null-Datenaufzeichnung mit der Messmethode 3 erfolgt. Die Stellung des Unterkiefers ist dabei unerheblich. Die Lage des Oberkiefers kann der Bissgabel, mit dem gehärteten Abdruckmaterial oder dem anderen Material, in Kombination mit der Null-Aufzeichnung entnommen werden.

Der Erfindung liegt **die Aufgabenstellung** zu Grunde, eine räumliche Beziehung zwischen Schädel und den Messdaten von Kiefermodellen, wie sie mit optischen Scannern gewonnen werden, bei geringerem Aufwand herzustellen.

Gemäß einem Aspekt wird die Aufgabe gelöst durch ein Verfahren zur Registrierung von 3D-Messdaten eines Kiefermodells, das ein Unterkiefermodell und ein Oberkiefermodell umfasst, in ein schädelbasisbezogenen Koordinatensystem. Das Verfahren umfasst:
Bestimmung einer ersten räumlichen Beziehung zwischen einem paraokklusalen Löffel oder einer Bissgabel und Kiefergelenken mittels eines computergestützten Registriersystems. Ferner umfasst das Verfahren das Bestimmung einer zweiten räumlichen Beziehung zwischen dem paraokklusalen Löffel oder der Bissgabel und dem Kiefermodell durch Verwenden lokalisierbarer Strukturen, deren räumliche Beziehung zum paraokklusalen Löffel oder zur Bissgabel bekannt ist. Des Weiteren umfasst das Verfahren das Bestimmen einer dritten räumlichen Beziehung zwischen Schädel und dem Kiefermodell in dem schädelbezogenen Koordinatensystem durch Verwenden der ersten und der zweiten räumlichen Beziehung.

Weitere Ausführungsformen des Verfahrens sind den davon abhängigen Ansprüchen zu entnehmen.

Gemäß einem weiteren Aspekt der Erfindung wird die Ausgabe gelöst durch eine Vorrichtung zur Registrierung von 3D-Messdaten eines Kiefermodells in ein schädelbasisbezogenen Koordinatensystem. Die Vorrichtung umfasst ein computergestütztes Registriersystem, das ausgebildet ist, erste Messdaten für eine Bestimmung einer ersten räumlichen Beziehung zwischen einem paraokklusalen Löffel oder einer Bissgabel und Kiefergelenken bereitzustellen. Ferner umfasst die Vorrichtung einen dentalen 3D-Scanner, der ausgebildet ist, zweite Messdaten für eine Bestimmung einer zweiten räumlichen Beziehung zwischen dem paraokklusalen Löffel oder der Bissgabel und dem Kiefermodell unter Verwendung lokalisierbarer Strukturen, deren räumliche Beziehung zum paraokklusalen Löffel oder zur Bissgabel bekannt ist, bereitzustellen. Des Weiteren umfasst die Vorrichtung eine Rechen- und Auswerteeinheit, die ausgebildet ist, eine dritte räumliche Beziehung zwischen Schädel und dem Kiefermodell in dem schädelbezogenen Koordinatensystem durch Verwenden der ersten und zweiten Messdaten zu bestimmen.

Weitere Ausführungsformen der Vorrichtung sind in den zugehörigen abhängigen Ansprüchen beschrieben.

Gemäß einem weiteren Aspekt der Erfindung wird die Aufgabe gelöst durch ein Passstück zur Verwendung in einer Vorrichtung, wie sie zuvor beschrieben ist, wobei das Passstück von einem 3D-Scanner lokalisierbare Strukturen bereit stellt.

Weitere Ausführungsformen des erfindungsgemäßen Passstücks sind auch den zugehörigen abhängigen Ansprüchen zu entnehmen.

### Ausführungsbeispiele erläutern und ergänzen die beanspruchte Erfindung.

- Abbildung 1: zeigt schematisch eine erfindungsgemäße Vorrichtung, wobei das Scannen mit aufgesteckter Bissgabel dargestellt ist,
- Abbildung 2: zeigt schematisch den Aufbau für Scan der Bissgabel in Form einer Draufsicht und entsprechenden Querschnittsansichten,
- Abbildung 3: zeigt schematisch eine Realisierung der Bissgabel nach einer anschaulichen Ausführungsform mit vestibulären lokalisierbaren Strukturen, wobei eine Draufsicht und entsprechende Querschnittsansichten dargestellt sind,
- Abbildung 4: zeigt schematisch eine Ausführungsform mit lingual lokalisierbaren Strukturen und Verbindung hinter dem letzten Backenzahn, wobei eine Draufsicht und entsprechende Querschnittsansichten dargestellt sind,
- Abbildung 5: zeigt schematisch eine weitere anschauliche Ausführungsform mit lingual lokalisierbaren Strukturen und frontaler Überbrückung, wobei eine Draufsicht und entsprechende Querschnittsansichten dargestellt sind.

Beispiele der Erfindung werden nunmehr mit Bezug zu den Abbildungen 1 bis 5 näher beschrieben.

Generell kann die Aufgabenstellung, nämlich, die räumliche Beziehung zwischen Schädel 20 und den Messdaten von Kiefermodellen, wie sie mit einem optischen Scanner 26 gewonnen werden, herzustellen, durch einen Scan des paraokklusalen Löffels bzw. der Bissgabel oder einer Abformung lokalisierbarer Strukturen gelöst werden.

Mittels der oben beschriebenen, computergestützten Analyse der Bewegungen wird eine erste räumliche Beziehung 22, die auch als die räumliche Beziehung I bezeichnet wird, zwischen den Kiefergelenken 4 und dem paraokklusalen Löffel bzw. Bissgabel 5 erfasst, und weiterhin mit einer im folgenden Absatz zu beschreibenden Methode eine zweite räumliche Beziehung 23, die auch als die räumliche Beziehung II bezeichnet wird, zwischen paraokklusalem Löffel 5 und Unterkiefer bzw. Unterkiefermodell 7 ermittelt. Durch Verknüpfung der beiden Beziehungen kann dann, in einer geeigneten Rechen- und Auswerteeinheit 27, eine dritte räumliche Beziehung, d.h. die räumliche Beziehung zwischen Kiefergelenken 4 und Unterkiefer berechnet werden. Die räumliche Beziehung zum Oberkiefer kann aus einem Bissregistrat entnommen werden. In Abbildung 1 wird der Ablauf schematisch dargestellt. Zur Veranschaulichung wurde im Schädel 20 ein einprojiziertes Unterkiefermodell 21 eingezeichnet. Anzumerken ist, dass generell ein Kiefermodell zumindest aus einem Unterkiefermodell und einen Oberkiefermodell, die hier stellvertretend beide mit dem Bezugszeichen 7 bezeichnet sind, aufgebaut ist, wobei der Einfachheit halber ein entsprechendes Oberkiefermodell in den Abbildungen nicht gezeigt ist.

Ein Lösungsansatz zur Ermittlung der räumlichen Beziehung zwischen paraokklusalen Löffel und Unterkiefer 23, etwa gemäß Anspruch 2, besteht im Verfahren "Scan des paraokklusalen Löffels". Der optische Scanner 26 scannt zunächst den auf das Unterkiefermodell 7 aufgesetzten paraokklusalen Löffel 5. Dieser wird dann entfernt, ohne das Unterkiefermodell 7 zu verschieben. In einem weiteren Schritt scannt der Scanner dann das Unterkiefermodell selbst. Weil das Unterkiefermodell nicht verschoben wurde, sind beide Scans im gleichen Koordinatensystem und die räumliche Beziehung II 23 ist erfasst.

Zur Bestimmung der räumlichen Beziehung zwischen Oberkiefer und Unterkiefer wird der Unterkiefer weiterhin nicht verschoben. Es wird ein Bissregistrat aufgesetzt, darauf der Oberkiefer (nicht gezeigt). In einem weiteren Schritt scannt der Scanner dann das so entstandene Unterkiefer-Oberkiefer-Paar. Weil das Unterkiefermodell nicht verschoben wurde, sind beide Scans im gleichen Koordinatensystem.

Das Bissregistrat ist einigen einfachen Fällen, wo ein geschlossener Biss besteht nicht zwingend erforderlich, weil die Bisssituation durch einfaches Zusammenfügen der beiden Kiefer in diesen Fällen in eindeutiger Weise gebildet wird. In anderen Fällen ist das Bissregistrat notwendig, also muss der zahnärztliche Prozess um das Nehmen eines Bissregistrats erweitert werden

Ein weiterer Lösungsansatz für die zugrunde liegende Aufgabenstellung besteht im Verfahren "Scan der Bissgabel". Bei diesem Lösungsansatz wird zunächst die räumliche Beziehung zwischen Oberkiefer und Bissgabel ermittelt, die auch als zweite räumliche Beziehung bezeichnet ist, danach die in den Ansprüchen als dritte räumliche Beziehung bezeichnete Beziehung zwischen Oberkiefer und Unterkiefer.

Zur Bestimmung der räumlichen Beziehung zwischen Oberkiefer und Bissgabel scannt der optische Scanner zunächst die auf dem Oberkiefermodell aufgesetzte Bissgabel. Diese wird dann entfernt, ohne das Oberkiefermodell zu verschieben. In einem weiteren Schritt scannt der Scanner dann das Oberkiefermodell selbst. Weil das Oberkiefermodell nicht verschoben wurde, sind beide Scans im gleichen Koordinatensystem beschrieben.

Zur Bestimmung der räumlichen Beziehung zwischen Oberkiefer und Unterkiefer wird der Oberkiefer weiterhin nicht verschoben. Es wird ein Bissregistrat aufgesetzt, darauf der Unterkiefer. In einem weiteren Schritt scannt der Scanner dann das so entstandene Unterkiefer-Oberkiefer-Paar. Weil das Oberkiefermodell nicht verschoben wurde, sind beide Scans im gleichen Koordinatensystem.

Das Bissregistrat ist einigen einfachen Fällen, wo ein geschlossener Biss besteht nicht zwingend erforderlich, weil die Bisssituation durch einfaches Zusammenfügen der beiden Kiefer in diesen Fällen in eindeutiger Weise gebildet wird. In anderen Fällen ist das Bissregistrat notwendig, also muss der zahnärztliche Prozess um das Nehmen eines Bissregistrats erweitert werden

Gegebenenfalls ist eine reduzierte Effizienz an den Verfahren "Scan des paraokklusalen Löffels" und "Scan der Bissgabel" zu beobachten, da typischerweise ein paraokklusaler Löffel oder eine Bissgabel nicht in Hinblick auf Scanfähigkeit konstruiert wurden. Bestimmte Werkstoffe, darunter Metall, sind für optische Scans nicht gut geeignet.

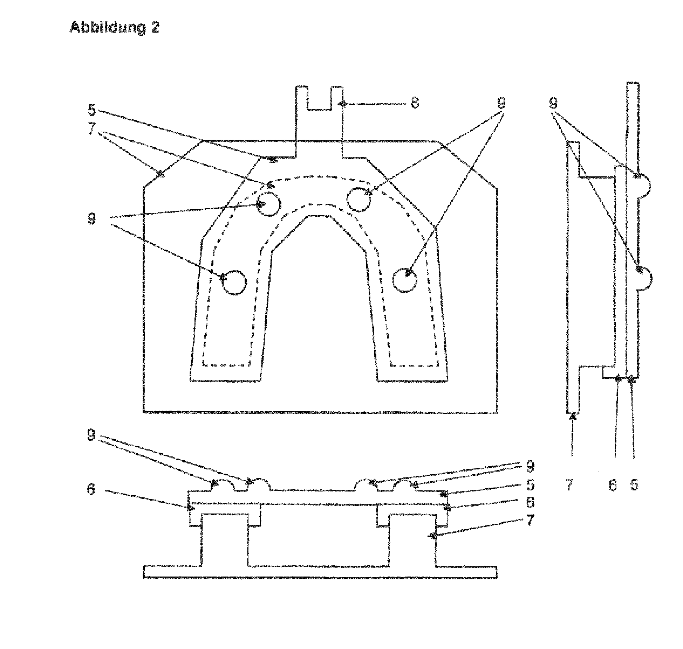

Das Problem kann mit Hilfe eines paraokklusalen Löffels bzw. einer Bissgabel gelöst werden, welche bestimmte okklusal angeordnete scanfähige, lokalisierbare Strukturen 9 aufweisen (Abbildung 2).

Durch diese Strukturen, welche durch den Scanner lokalisierbar sind, kann die Lage des paraokklusalen Löffels bzw. der Bissgabel in allen räumlichen Freiheitsgraden und Winkelfreiheitsgraden erfasst werden kann. Man kann z.B. matte weiße Halbkugeln an einem paraokklusalen Löffel oder auf der Oberseite der Bissgabel integrieren. Drei Halbkugeln sind zur Feststellung der Lage ausreichend, ab vier Halbkugeln wäre die Bestimmung redundant und damit genauer. In allen Abbildungen werden vier Halbkugeln als Beispiel für lokalisierbare Strukturen 9, 12, 13 dargestellt, wobei jedoch auch eine andere Anzahl verwendet werden kann.

Die zuvor beschriebenen Ausführungsformen lösen die Aufgabe, haben aber Verbesserungspotential im Hinblick auf folgende Aspekte.
- Die lokalisierbaren Strukturen haben Patientenkontakt. Sie stören den Patienten bei Kaubewegungen. Sie müssen im Allgemeinen kleiner ausgeführt werden, als wenn der Patient bei der Formgebung nicht zu berücksichtigen wäre. Größere Lokalisationsstrukturen jedoch erlauben eine genauere Lokalisation, insbesondere der Winkelfreiheitsgrade.
- Der bei den Ärzten etablierte Prozess muss verändert werden. Die dort gelagerten paraokklusalen Löffel bzw. Bissgabeln brauchen nicht durch neue, modifizierte Pendants ersetzt zu werden.

In Fortentwicklung des Gedankens kann man die lokalisierbaren Strukturen erst beim Scan in einer definierten und reproduzierbaren Art und Weise auf den paraokklusalen Löffel bzw. die Bissgabel aufsetzen. Dazu kann ein passender Aufstecker mit Formschluss zum paraokklusalen Löffel bzw. zur Bissgabel dienen.

Nachteilig an dieser Lösung wäre jedoch die Tatsache, dass ggf. eine Bissgabel Platz in okklusaler Richtung benötigt. Dadurch wird ein größerer Tiefenmessbereich des Scanners nötig, als wenn nur das Kiefermodell alleine vermessen würde. Weiterhin benötigt sowohl ein paraokklusaler Löffel als auch eine Bissgabel Platz in frontaler Richtung. Durch beide Tatsachen wird der Bau eines größeren Scanners notwendig.

Es ist vorteilhaft, ein besonderes Passstück bereit zu stellen, welches diesen Nachteil vermeiden kann:
In einer vorteilhaften Ausführungsform des Passstücks, hat das Passstück folgende Eigenschaften
   - Es ist aus scanfähigem Material hergestellt (scanfähiges Passstück 13).
   - Es weist einen Formschluss 18 zum paraokklusalen Löffel bzw. zur Bissgabel auf. Dadurch ist die räumliche Beziehung mit geringen Toleranzen mechanisch reproduzierbar. Die räumliche Beziehung zum paraokklusalen Löffel bzw. zur Bissgabel ist aufgrund der Konstruktion des Passstücks bekannt.
   - Es weist lokalisierbare Strukturen auf.
   - Es wird mittels eines Verbindungsmaterials, vorzugsweise Gips, mit dem Unterkiefermodell bzw. dem Oberkiefermodell fest verbunden und verbleibt während des Scans am Modell, während hingegen der paraokklusale Löffel bzw. die Bissgabel entfernt werden.

Die Transformation zwischen Kiefergelenken und Unterkiefer bzw. Oberkiefer wird berechnet aus der Lage der lokalisierbaren Strukturen relativ zum scanfähigen Passstück und der Lage des scanfähigen Passstücks relativ zum paraokklusalen Löffel bzw. zur Bissgabel.

Diese Ausführungsform weist keinen der bisher aufgezählten Nachteile auf. Sie hat ferner den Vorteil, dass das scanfähige Passstück keinen Patientenkontakt hat. Dies vereinfacht die Einführung der Technologie und erlaubt eine größere Auswahl von Materialien.

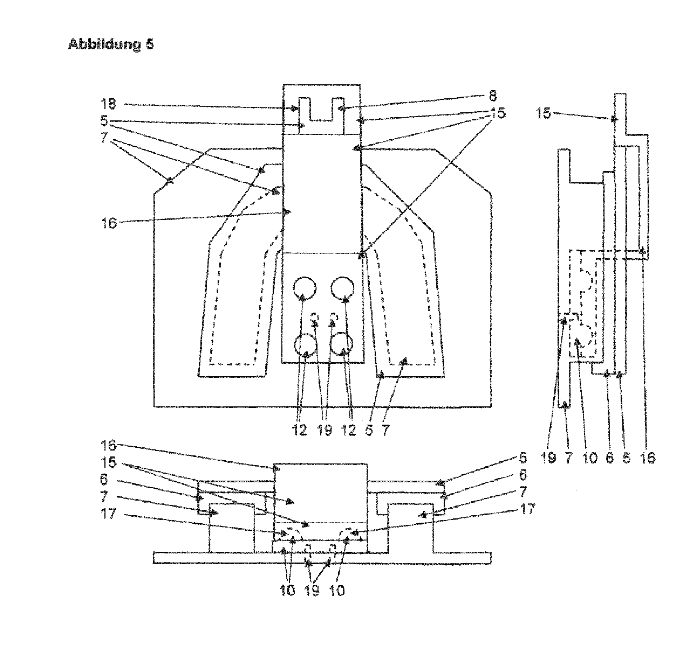

Eine weitere Ausführungsform des Passstücks (Abbildung 5) verwendet ein "Abform-Passstück" 15. Das Verfahren mit "Abform-Passstück" läuft ähnlich dem Verfahren mit scanfähiges Passstück 13 in der zuvor beschriebenen Ausführungsform ab, jedoch werden seine Strukturen in einem Verbindungsmaterial, vorzugsweise Gips 10, abgeformt. Es verbleibt nicht während des Scans am Modell, sondern wird, gemeinsam mit dem paraokklusalen Löffel bzw. der Bissgabel, vorher entfernt. Die im Verbindungsmaterial vorzufindende Abformung der lokalisierbaren Strukturen 17 wird gescannt.

Die Berechnung der (zweiten) räumlichen Beziehung zwischen Kiefergelenken und Unterkiefer bzw. Oberkiefer geschieht mit Hilfe der aus der Konstruktion des Abform-Passstücks bekannten räumlichen Beziehung der Abformung der Lokalisierungsstrukturen relativ zur paraokklusalen Löffel bzw. zur Bissgabel.

Die Ausführungsform mit Abformung hat gegenüber der Ausführungsform mit scanfähigem Passstück folgende Vorteile
- Das optische 3D-Scannen von Gips ist mit höherer Genauigkeit möglich, als das von anderen Oberflächen. Somit wird man bei der Wahl des Materials für das Passstück frei, und kann auf Materialien mit schlechteren Eigenschaften bzgl. des 3D-Scans einsetzen, wie z.B. blankes Aluminium.
- Das Passstück ist wieder verwendbar, es kann in geringerer Stückzahl produziert werden, es kann deswegen aus hochwertigeren und steiferen Materialien produziert werden. Dadurch werden Fehler durch Verformung des Passstücks vermieden.

Das Verbindungsmaterial mit der Abformung 17 kann zudem gegen die Modellplatte mit einer Verstiftung 19 versehen werden. Dies hat den Vorteil, dass die Abformung entnommen werden kann, wenn sie nicht benötigt wird.

### Formschluss

Der Adapter zur unterkieferbezogenen Komponente ist bereits auf Formschluss zum Unterkieferbogen hin konstruiert. Es ist vorteilhaft, dieses Konstruktionsmerkmal zu nutzen und den Formschluss für das Passstück an dieser Stelle vorzusehen.

### Anordnung der lokalisierbaren Strukturen

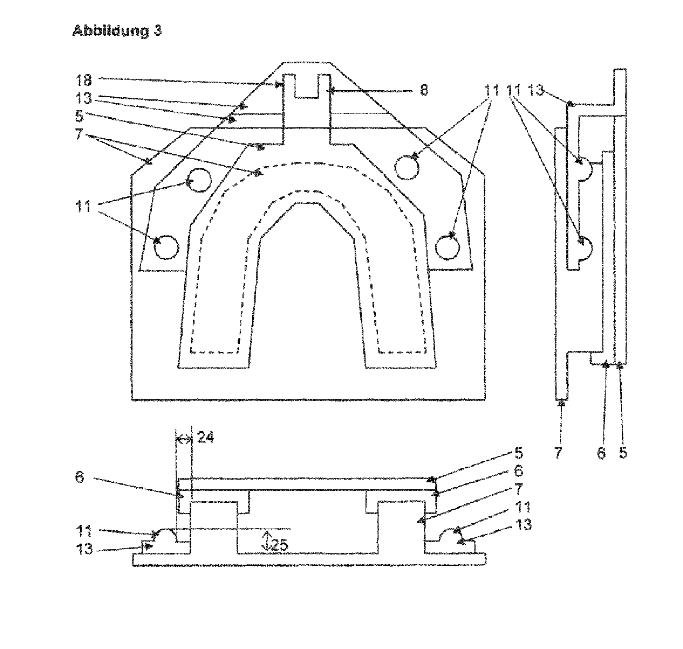

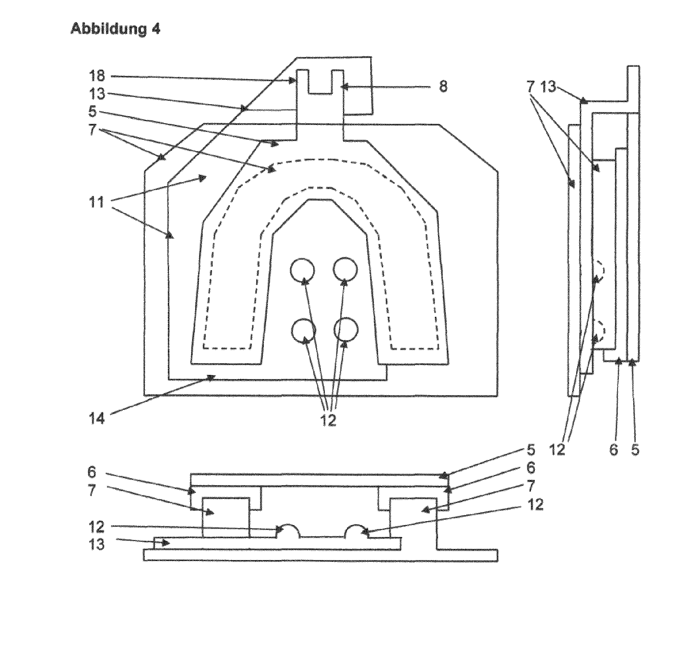

Es gibt zwei Möglichkeiten zur räumlichen Anordnung der Strukturen relativ zur Zahnreihe des Unterkiefers bzw. Oberkiefers: Anordnung auf der vestibulären Seite oder auf der lingualen Seite. Entsprechend sind vestibulär angeordnete lokalisierbare Strukturen 12 (Abbildung 3) oder lingual angeordnete lokalisierbare Strukturen 13 (Abbildung 4) zu unterscheiden.

Besonders vorteilhaft ist die Verwendung der lingualen Seite: Die lokalisierbaren Strukturen können in der Mitte des Kieferbogens Platz finden und dort relativ groß ausgeführt werden. Auf der vestibulären Seite hingegen endet in der Regel das Messfeld des Scanners in geringem Abstand von der Zahnreihe.

Die Lokalisierungsstrukturen sollen den Scanprozess nicht behindern. Daher müssen die Lokalisierungsstrukturen in ausreichendem Abstand 24 von der Zahnreihe angebracht werden und eine geringe Höhe 25 aufweisen, der zufolge beim Scannen die Lokalisierungsstrukturen keinen Schatten auf die Zahnreihe werfen.

Eine Passstück, welches den Formschluss zum paraokklusalen Löffel oder zur Bissgabel auf der vestibulären Seite bewerkstelligt und die Strukturen auf der lingualen Seite, kann eine frontale Überbrückung 16 (Abbildung 5) aufweisen. Damit die Brückenkonstruktion beim Scannen nicht behindert, muss hier die Ausführungsform mit Abformung verwendet werden.

Alternativ kann eine Verbindung hinter dem letzten Backenzahn 14 sowohl mit der Ausführungsform mit scanfähigem Passstück als auch mit der Ausführungsform mit Abformung realisiert werden. (Abbildung 4).

### Klarstellung bzgl. Kaubewegungen und virtueller Artikulator

Ein computergestütztes Registriersystem ist nicht nur in der Lage die (erste) räumliche Beziehung zwischen Unterkiefer und schädelbezogener Komponente in Bissstellung zu ermitteln, sondern auch in jeder anderen Stellung währen einer dynamischen Kieferbewegung, z.B. einer Kaubewegung. Die genannten Überlegungen zur Bestimmung der räumlichen Beziehungen gelten sowohl für die Biss-Situation als auch für jeden anderen Zeitpunkt einer dynamischen Kieferbewegung.

### Liste der in den Zeichnungen verwendeten Begriffe mit Bezugszeichen

- 1: schädelbezogenen Komponente
- 2: unterkieferbezogene Komponente
- 3: Messmethode
- 4: Lage der Kiefergelenke
- 5: Bissgabel

- 6: Abdruckmasse
- 7: Modell des Unterkiefers mit Modellplatte
- 8: Adapter zur unterkieferbezogenen Komponente
- 9: okklusal angeordnete lokalisierbare Strukturen
- 10: Verbindungsmaterial, vorzugsweise Gips

- 11: vestibulär angeordnete lokalisierbare Strukturen
- 12: lingual angeordnete lokalisierbare Strukturen
- 13: scanfähiges Passstück
- 14: Verbindung hinter dem letzten Backenzahn
- 15: Abform-Passstück

- 16: frontale Überbrückung
- 17: Abformung der lokalisierbaren Strukturen
- 18: Formschluss
- 19: Verstiftung des Verbindungsmaterials mit der Modellplatte
- 20: Schädel

- 21: Einprojiziertes Modell des Unterkiefers mit lokalisierbaren Strukturen
- 22: Räumliche Beziehung I zwischen Kiefergelenken und paraokklusalem Löffel
- 23: Räumliche Beziehung II zwischen paraokklusalem Löffel und Unterkiefer
- 24: Ausreichender Abstand
- 25: Geringe Höhe
- 26: 3D-Scanner
- 27: Rechen- und Auswerteeinheit
- 28: Computergestütztes Registriersystem

## Patentansprüche

1. Verfahren zur Registrierung von 3D-Messdaten eines Kiefermodells, das ein Unterkiefermodell (7) und ein Oberkiefermodell (7) umfasst, in ein schädelbasisbezogenen Koordinatensystem, wobei das Verfahren umfasst
- Bestimmung einer ersten räumlichen Beziehung (22) zwischen einem paraokklusalen Löffel (5) oder einer Bissgabel (5) und Kiefergelenken (4) mittels eines computergestützten Registriersystems (28),
- Bestimmung einer zweiten räumlichen Beziehung (23) zwischen dem paraokklusalem Löffel (5) oder der Bissgabel (5) und dem Kiefermodell (7) durch Verwenden lokalisierbarer Strukturen (9), deren räumliche Beziehung zum paraokklusalen Löffel (5) oder zur Bissgabel (5) bekannt ist und
- Bestimmen einer dritten räumlichen Beziehung zwischen Schädel (20) und dem Kiefermodell (7) in dem schädelbezogenen Koordinatensystem durch Verwenden der ersten (22) und der zweiten (23) räumlichen Beziehung.

2. Verfahren nach Anspruch 1, wobei Bestimmen der zweiten räumlichen Beziehung (23) umfasst:
- Modifizierung des Unterkiefermodells (7) durch Anbringen eines scanfähigen Passstücks (13), welches die lokalisierbaren Strukturen (9) aufweist, mit Formschluss (8) am paraokklusalen Löffel (5),
- Herstellen einer Verbindung zwischen dem Unterkiefermodell (7) und dem scanfähigen Passstück (13),
- Entfernen des paraokklusalen Löffels (5),
- Scannen des modifizierten Unterkiefermodells (7),
- Ermittlung einer räumlichen Beziehung zwischen Messdaten des Unterkiefermodells(7) und des Passstücks (13) als die zweite räumliche Beziehung,
wobei Bestimmen der dritten räumlichen Beziehung umfasst:
Berechnen der räumlichen Beziehung zwischen Schädel (20) und den Messdaten des Unterkiefermodells (7) aus
- der ersten räumlichen Beziehung (22) zwischen dem paraokklusalen Löffel (5) und den Kiefergelenken (4),
- einer aufgrund der Konstruktion des scanfähigen Passstücks (13) bekannten räumlichen Beziehung zwischen dem paraokklusalen Löffel (5) und dem scanfähigen Passstück (13) und
- der ermittelten räumlichen Beziehung zwischen den Messdaten des Unterkiefermodells (7) und dem scanfähigen Passstück (13);
und Ermitteln einer räumlichen Beziehung zwischen dem Oberkiefermodell (7) und dem Unterkiefermodell (7) durch Scan der beiden in Bisssituation zusammengefügten Modelle (7, 7).

3. Verfahren nach Anspruch 1, wobei Bestimmen der zweiten räumlichen Beziehung umfasst:
- Modifizierung des Oberkiefermodells (7) durch Anbringen eines scanfähigen Passstücks (13), welches die lokalisierbaren Strukturen aufweist (9), mit Formschluss an der Bissgabel (5),
- Herstellen einer Verbindung zwischen dem Oberkiefermodell (7) und dem scanfähigen Passstück (13),
- Entfernen der Bissgabel (5),
- Scannen des modifizierten Oberkiefermodells (7),
- Ermittlung einer räumlichen Beziehung zwischen Messdaten des Oberkiefermodells (7) und dem scanfähigen Passstück (13) als die zweite räumliche Beziehung,
wobei Bestimmen der dritten räumlichen Beziehung umfasst:
- Berechnen einer räumlichen Beziehung zwischen Schädel (20) und den Messdaten des Oberkiefermodells (7) aus der
- ersten räumlichen Beziehung (22) zwischen den Kiefergelenken (4) und der Bissgabel (5),
- einer aus der Konstruktion des scanfähigen Passstücks (13) bekannten räumlichen Beziehung zwischen der Bissgabel (5) und dem scanfähigen Passstück (13),
- und der ermittelten zweiten räumlichen Beziehung zwischen den Messdaten des Oberkiefermodells (7) und dem scanfähigen Passstück (13),
- Ermitteln einer räumlichen Beziehung zwischen dem Oberkiefermodell und dem Unterkiefermodell durch Scan der beiden in Bisssituation zusammengefügten Modelle.

4. Verfahren nach Anspruch 1, wobei Bestimmen der zweiten räumlichen Beziehung umfasst:
- Modifizierung eines Unterkiefermodells (7) durch Anbringen eines Abform-Passstücks (15), welches die lokalisierbaren Strukturen (9) aufweist, mit Formschluss an dem paraokklusalen Löffel (5),
- Abformung der lokalisierbaren Strukturen (9) des Abform-Passstücks (15) auf das Unterkiefermodell,
- Entfernen des paraokklusalen Löffels (5) und des Abform-Passstücks (15),
- Scannen des modifizierten Unterkiefermodells (7),
- Ermittlung einer räumlichen Beziehung zwischen Messdaten des Kiefermodells (7) und dem Abform-Passstück (15) durch Messung von durch das Abform-Passstück (15) abgeformten Strukturen (17) als die zweite räumliche Beziehung,
wobei Bestimmen der dritten räumlichen Beziehung umfasst:
- Berechnen einer räumlichen Beziehung zwischen Schädel (20) und den Messdaten des Unterkiefermodells aus
- der ersten räumlichen Beziehung zwischen den Kiefergelenken (4) und dem paraokklusalen Löffel (5),
- einer aufgrund der Konstruktion des Abform-Passstücks (15) bekannten räumlichen Beziehung zwischen dem paraokklusalen Löffel (5) und dem Abform-Passstück (15) und
- der ermittelten räumlichen Beziehung zwischen den Messdaten des Unterkiefermodells und dem Abform-Passstück,
- Ermitteln einer räumlichen Beziehung zwischen dem Oberkiefermodell und dem Unterkiefermodell durch Scan der beiden in Bisssituation zusammengefügten Modelle.

5. Verfahren nach Anspruch 1, wobei Ermitteln der zweiten räumlichen Beziehung umfasst:
- Modifizierung des Oberkiefermodells (7) durch Anbringen eines Abform-Passstücks (15), welches die lokalisierbaren Strukturen (9) aufweist, mit Formschluss an der Bissgabel (5)
- Abformung der lokalisierbaren Strukturen (9) des Abform-Passstücks (15) auf das Oberkiefermodell (7),
- Entfernen der Bissgabel (5) und des Abform-Passstücks (15),
- Scannen des modifizierten Oberkiefermodells,
- Ermittlung einer räumlichen Beziehung zwischen Messdaten des Oberkiefermodells und der Abformung der lokalisierbaren Strukturen (17) als die zweite räumliche Beziehung,
wobei Bestimmen der dritten räumlichen Beziehung umfasst:
- Berechnen einer räumlichen Beziehung zwischen Schädel (20) und den Messdaten des Unterkiefermodells aus
- der ersten räumlichen Beziehung zwischen den Kiefergelenken (4) und der Bissgabel (5),
- einer aufgrund der Konstruktion des Abform-Passstücks (15) bekannten räumlichen Beziehung zwischen der Bissgabel (5) und dem Abform-Passstück (15) und
- der zweiten räumlichen Beziehung ,
- Ermitteln einer räumlichen Beziehung zwischen dem Oberkiefermodell und dem Unterkiefermodell durch Scan der beiden in Bisssituation zusammengefügten Modelle.

6. Vorrichtung zur Registrierung von 3D-Messdaten eines Kiefermodells in ein schädelbasisbezogenen Koordinatensystem, wobei die Vorrichtung umfasst:
ein computergestütztes Registriersystem (28), das ausgebildet ist, erste Messdaten für eine Bestimmung einer ersten räumlichen Beziehung (22) zwischen einem paraokklusalen Löffel (5) oder einer Bissgabel (5) und Kiefergelenken (4) bereitzustellen,
einen dentalen 3D-Scanner (26), der ausgebildet ist, zweite Messdaten für eine Bestimmung einer zweiten räumlichen Beziehung (23) zwischen dem paraokklusalen Löffel (5) oder der Bissgabel (5) und dem Kiefermodell (7) unter Verwendung lokalisierbarer Strukturen (9), deren räumliche Beziehung zum paraokklusalen Löffel (5) oder zur Bissgabel (5) bekannt ist, bereitzustellen, und
eine Rechen- und Auswerteeinheit (27), die ausgebildet ist, eine dritte räumliche Beziehung zwischen Schädel (20) und dem Kiefermodell (7) in dem schädelbezogenen Koordinatensystem durch Verwenden der ersten und zweiten Messdaten zu bestimmen.

7. Vorrichtung nach Anspruch 6, die ferner ein scanfähiges Passstück (13) umfasst, das die lokalisierbaren Strukturen aufweist und mit Formschluss an dem paraokklusalem Löffel anbringbar ist.

8. Vorrichtung nach Anspruch 6, das ferner ein scanfähiges Passstück (13) umfasst, das die lokalisierbaren Strukturen aufweist und mit Formschluss an der Bissgabel anbringbar ist.

9. Vorrichtung nach Anspruch 6, das ferner ein scanfähiges Passstück (15) umfasst, das die lokalisierbaren Strukturen aufweist und mit Formschluss an dem paraokklusalen Löffel anbringbar ist und zur Abformung der lokalisierbaren Strukturen auf ein Unterkiefermodell ausgebildet ist.

10. Vorrichtung nach Anspruch 6, das ferner ein scanfähiges Passstück (15) umfasst, das die lokalisierbaren Strukturen aufweist und mit Formschluss an der Bissgabel anbringbar ist und zur Abformung der lokalisierbaren Strukturen auf ein Oberkiefermodell ausgebildet ist.

11. Passstück zur Verwendung in einer Vorrichtung zur Registrierung von 3D-Messdaten eines Kiefermodells in ein schädelbasisbezogenen Koordinatensystem, wobei das Passstück (13; 15) zur Bereitstellung von für einen 3D-Scanner (26) lokalisierbare Strukturen (9; 17) ausgebildet ist.

12. Passstück nach Anspruch 11, das ferner so ausgebildet ist, dass es mit Formschluss an einem paraokklusalem Löffel anbringbar ist.

13. Passstück nach Anspruch 11, das ferner so ausgebildet ist, dass es mit Formschluss an einer Bissgabel anbringbar ist.

14. Passstück nach Anspruch 11, das mit Formschluss an einem paraokklusalen Löffel anbringbar und zur Abformung der lokalisierbaren Strukturen auf ein Unterkiefermodell ausgebildet ist.

15. Passstück nach Anspruch 11, das mit Formschluss an einer Bissgabel anbringbar und zur Abformung der lokalisierbaren Strukturen auf ein Oberkiefermodell ausgebildet ist.
